# EUROPEAN PATENT SPECIFICATION

(11) **EP 0 351 421 B1**
(45) Date of publication and mention of the grant of the patent: **19.05.1993**
(21) Application number: 88908851.4
(22) Date of filing: 09.09.1988
(51) Int. Cl.: A61M 39/00

(54) **ONE-WAY VALVE**
RÜCKSCHLAGVENTIL
SOUPAPE A UNE VOIE

(30) Priority: 16.09.1987 US 97181; 31.08.1988 US 237849
(43) Date of publication of application: 24.01.1990
(73) Proprietor: Uresil L.P., Skokie, Illinois 60077 (US)
(72) Inventor: MELINYSHYN, Lev, Mt. Prospect, Illinois 60056 (US); GOLDBERG, Edward M., Glencoe, Illinois 60077 (US)
(74) Representative: Allden, Thomas Stanley
(86) International application number: US8803112
(87) International publication number: WO8902291

(56) References cited:
- AU-A- 420 173
- BE-A- 872 870
- DE-A- 2 616 290
- FR-A- 1 468 343
- FR-A- 2 585 105
- US-A- 3 417 750

## Description

This invention relates to a one-way valve.

The term "one-way valve" is intended to mean a device which allows the passage of substances through it in one direction only. Such valves are used in a host of applications, including medical applications, where fluids must be withdrawn from body activities without reflux to the cavities.

It is most desirable to be able to achieve low "crack" resistance in one-way valves, so that the valves will open in the desired direction of flow on the application of minimal pressure. Similarly, it is most desirable to maximize reflux sensitivity of such valves, so that they will close quickly to prevent back-flow through the valve. It is likewise important that the valves continue to operate when particulate matter becomes lodged in the valves.

In many applications requiring one-way valves, it is important that the valves be compact and flexible, so that they do not occupy excessive space in the devices in which they are employed.

Finally, it is often important that the valves have a long shelf life, so that they will be reliable whenever the devices in which they are employed are put to use. In addition, for medical applications, the valves must be constructed from materials approved for use in the treatment of human objects, and they often must be able to stand up to drastic pressure changes and to a lesser degree to temperature and humidity changes, all as part of sterilization procedures.

In AU-A-420173 there is generally disclosed a one-way valve comprising two flat resilient members in face-to-face relationship with each other, the resilient members being bonded along two generally parallel tracks defining a passageway between, an inlet port communicating with one end of the passageway, and an outlet port communicating with the opposite end of the passage, the passageway allowing fluid flow therethrough in a direction from the inlet port to the outlet port but closing in response to the application of a suction force to the inlet port.

More particularly, the inner edges of the tracks in AU-A-420173 which define the passageway are straight, and we have found that with a passageway defined by straight inner edges, when a high suction force is applied to the inlet port that there is a tendency for fluid to leak back along the straight inner edges of the tracks towards the inlet port through the "closed passageway".

In accordance with the present invention as claimed, the aforesaid generally disclosed one-way valve is characterised in that the tracks have a tortuous profile along their inner edges defining the passageway.

The advantage of the invention is that the tortuous profile is found to prevent a reflux flow of fluid along the inner edges of the tracks when a high suction force is applied to the inlet port. This characteristic is most important when the one-way valve is used in medical applications to drain liquid and gases from the chest since it is imperative that the valve permits escape of such fluids without reflux.

Preferably, the tortuous profile comprises a sawtooth pattern with the or each sawtooth projecting from each of the tracks into the passageway and including a leading edge directed towards the outlet port and a trailing edge towards the inlet port.

Advantageously, the resilient members are made from a material chosen from the group consisting of polyethylene, mylar, nylon and polyvinyl chloride.

In order that the present invention may be well understood there will now be described some embodiments thereof, given by way of example, reference being made to the accompanying drawings, in which:
Figure 1 is a plan view of a one-way valve embodying the present invention;
Figure 2 is a cross-sectional view of the valve of Figure 1, taken along line 2-2 thereof;
Figure 3 is a cross-sectional view of the valve of Figure 1, taken along line 3-3 thereof;
FIGURE 4 is a plan view of the valve of FIG. 1 illustrating the flow of fluid therethrough;
FIGURE 5 is a cross-sectional view of the valve of FIG. 4, taken along line 5-5 thereof;
FIGURE 6 is a plan view of a one-way valve lacking the tortuous passageway profile of the present invention;
FIGURE 7 is a cross-sectional view of the valve of FIG. 6, taken along line 7-7 thereof;
FIGURE 8 is an alternative one-way valve embodying the present invention which includes two passageways;
FIGURE 9 is a plan view of the one-way valve of FIG. 1 in which silicone oil has been introduced between the opposing faces of the resilient members;
FIGURE 10 is a side view of the valve of FIG. 9, in which the valve has been creased across the passageway; and
FIGURE 11 is a side view of the valve of FIG. 10 after the folded valve is released.

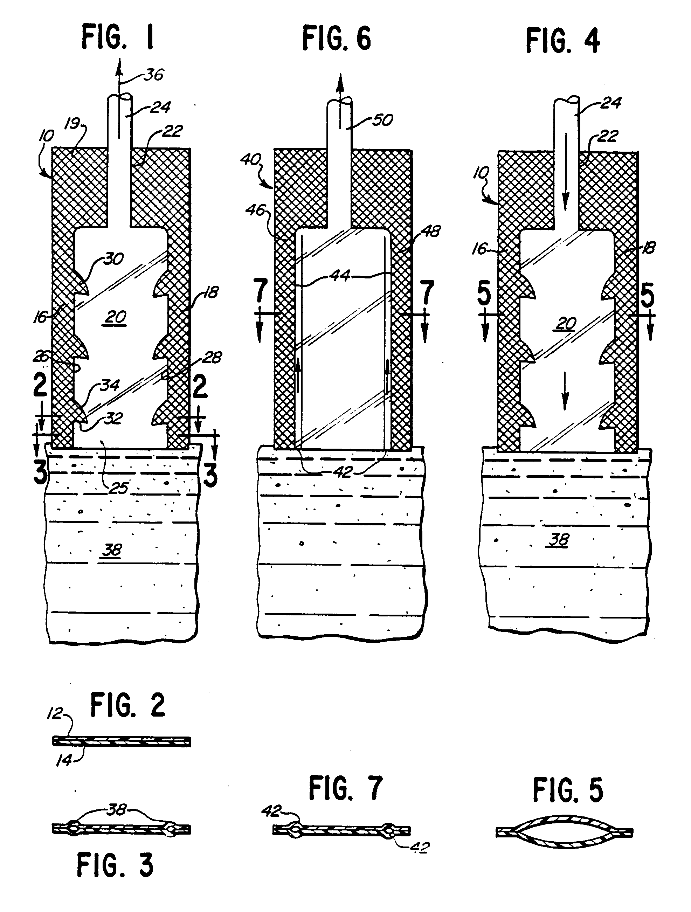

Referring first to FIGURES 1-3, a one-way valve 10 is illustrated, including flat resilient members 12 and 14 in face-to-face relationship with each other. The flat resilient members 12 and 14 are bonded along generally parallel tracks 16 and 18 which define a passageway 20 therebetween.

The tracks 16 and 18 come together near the top of the valve 19 to define an inlet port 22 which is narrower than the passageway 20. The inlet port 22 is sealingly attached to a rigid conduit 24. The valve 10 is also provided with an outlet port 25 at the opposite end of the passageway 20.

The tracks 16 and 18 each have a tortuous profile along their respective inner edges 26 and 28. In the embodiment illustrated, the tortuous profile comprises a series of sawteeth 30 projecting from each of the track inner edges into the passageway 20. Each sawtooth 30 has a leading edge 32 and a trailing edge 34. The sawteeth 30 are positioned with their leading edges 32 oriented toward the outlet port 25 and their trailing edges 34 oriented towards the inlet port 22. In an alternative, somewhat less desirable embodiment a single sawtooth may be used at each of the track inner edges.

The tortuous profile may take various different forms which create twists, turns, curves and windings along the outer edges of passageway 20 to prevent reflux flow along the outer edges of the valve passageway, as explained below.

When a suction force is applied to the conduit 24, as indicated by the arrow 36, the valve 10 takes on the respective cross-sectional profiles illustrated in FIGURES 2 and 3. Thus, any fluid 38, which is drawn up along the initial generally straight edges of the passageway 20 at outlet port 25 (FIG. 3) will not pass through the valve 10, as explained below in connection with the discussion regarding FIGS. 6 and 7.

The resilient members 12 and 14 may be made from a wide variety of resilient materials including for example, polyethylene, mylar, nylon and polyvinyl chloride. All of these materials are approved for use in the treatment of human subjects. The resilient members should be from about 1 to about 10 mm in thickness, although it is preferred that the resilient members be from about 3 to about 5 mm thick. In a particularly preferred embodiment, the resilient members will be about 3 mm in thickness.

The resilient members 12 and 14 may be bonded to each other and to the rigid conduit 24 by any conventionally available means which would not unduly restrict the operation of the valve 10. It is preferred that the resilient members 12 and 14 be bonded by a heat sealing technique such as thermal impulse heating or hot bar heating. Among presently available bonding techniques, thermal impulse heating has been found to be particularly desirable. The temperatures, pressures and other parameters used in bonding the resilient members 12 and 14 will depend upon the material of the resilient members, their thickness, the length and width of the valve 10, and the desired valve crack resistance and reflux sensitivity.

In one preferred embodiment in which a heat-sealing bonding method is used, the resilient members 12 and 14 are laminates comprising a heat-sealable layer and a thermally-resistant layer. In this embodiment, the thermally-resistant layer primarily governs the physical properties of the valve 10 while the heat-sealable layer bonds the resilient members 12 and 14. These laminated resilient member 12 and 14 are disposed in the valves 10 with their heat-sealable layers in face-to-face relationship. Thus, the heat-sealable layers will melt and adhere during the bonding process at a bonding temperature which will not significantly alter the resilience, integrity, and other necessary and desirable characteristics of the thermally-resistant layer.

The laminated resilient layers may be made by any known means, including conventional lamination and coextrusion techniques. Also, where a laminated material is used, the heat-sealable layer should be from about 2.5 to about 3.5 mm in thickness and the thermally-resistant layer should be from about 0.50 to about 0.75 mm in thickness. In a particularly preferred embodiment, the heat-sealable layer should be about 3.0 mm in thickness and the thermally-resistant layer should be about 0.75 mm in thickness.

The heat-sealable layer of the laminated resilient members 12 and 14 may be chosen from the group consisting of low density polyethylene and ethyl vinyl acetate. The thermally-resistant layer may be chosen from the group consisting of nylon, mylar and linear low density polyethylene. A particularly preferred laminated material, comprising low density polyethylene and nylon, can be made by laminating low density polyethylene to Capran (Registered Trade Mark) film available from Allied Engineered Plastics of Mooristown, New Jersey. This laminated material takes on certain desirable characteristics of the nylon including outstanding resistance to puncture, abrasion and flex cracking, as well as high burst and impact strength and high tensile and tear strength.

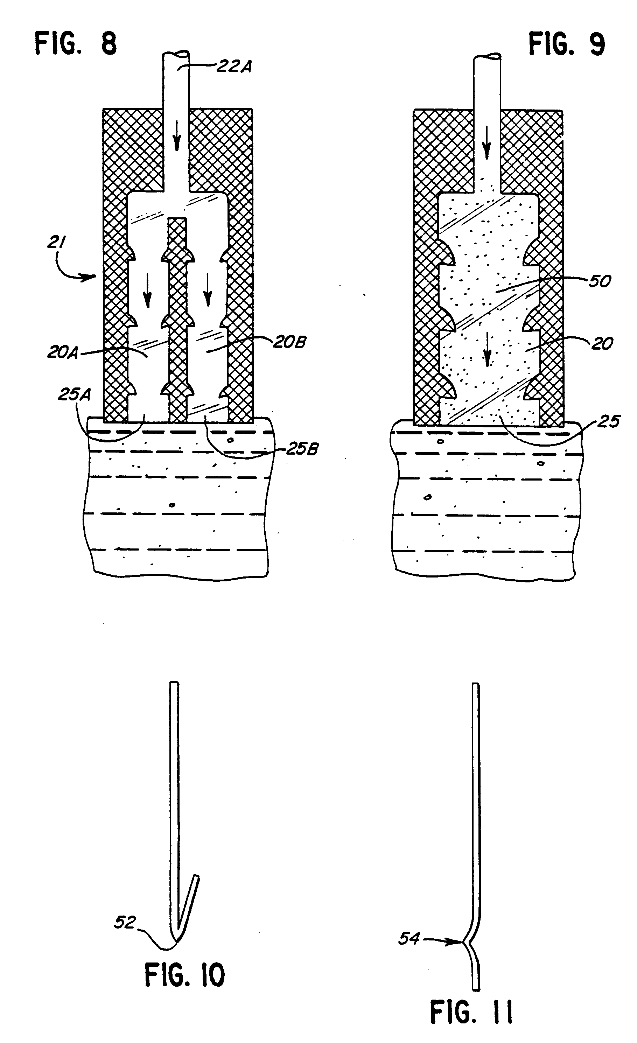

Although the valve 10 illustrated in FIG. 1 shows a single pair of tracks 16 and 18 and a single passageway 20, as illustrated in FIGURE 8, a one-way valve 21 in accordance with the present invention may have two or more pairs of sealing tracks defining a plurality of passageways 20A and 20B with the passageways having a single common inlet 22A and a plurality of independent outlet ports 25A and 25B. In addition, one-way valves in accordance with the present invention may be made in various lengths and widths to tailor the valve properties to the desired applications.

The valve 10 illustrated in FIG. 1 can be used in medical applications, for example, to drain fluid and gases from the chest, where it is essential that the liquids and gases be permitted to escape without reflux. In this application, it is important that the valve prevent blood clots and other solids expelled from the chest cavity from causing valve failure.

Thus, the rigid conduit 24 would be connected to a drain placed in the chest cavity (not shown). As illustrated in FIG. 4, gases and fluids expelled from the chest cavity flow through the conduit 24, inlet port 22 and passageway 20 into a receptacle (not shown) containing liquid 38. When a suction force is applied to the conduit 24, the valve 10 closes, as illustrated in FIGS. 1-3, to prevent liquids and gases from being drawn back up through the valve and into the chest cavity. Due to the design of the valve 10, blood clots and other solids which may be introduced into the passageway 20 during the draining procedure are held in place and will not interfere with the operation of the valve. The opposing faces of the resilient members 16 and 18 simply seal above and below such solids without hindering the valve function.

While the invention is not intended to be limited by any particular theory of the operation of the valve 10, in the absence of a tortuous profile, as in the case of the valve 40 illustrated in FIGURES 6 and 7, fluid leakage results under high suction forces. Apparently, such leakage occurs due to the formation of small channels 42 along the inner edges 44 of tracks 46 and 48 which permit fluids (liquids or gases) to travel up the valve when suction is applied to conduit 50.

The tortuous profile in the valve 10 (FIGURE 1) interrupts the channels which otherwise form along the outer edges of the valve passageway 20, redirecting the flow toward the centre of the passageway where it is effectively blocked. The tortuous profile is also believed to maintain the opposing faces of the resilient members 12 and 14 in enhanced intimate contact to improve the reflux sensitivity of the valve 10.

In another preferred embodiment of the invention, illustrated in FIGURES 9-11, a wetting agent 50 is introduced into the passageway 20 to "wet" the inner opposing faces of the resilient members of the valve 21. Wetting agents have been found to improve the reflux sensitivity of the valve. In addition, although the operation of the valve may be impaired when it is subjected to pressure, temperature and humidity changes, as in, for example, a sterilization procedure, addition of a high boiling liquid as the wetting agent will maintain or restore the valve function.

The principal criteria in choosing the wetting agent are that it will not degrade or damage the valve material, that it will not boil off, that it will not deposit particulate matter in the valve, and that it will not cause the opposing faces of the valve to permanently adhere to each other. Oils meeting the above criteria are the preferred agents. A particularly preferred wetting agent is silicone oil, because of its high boiling point and wetting properties and also because it is a material approved for use in medical devices.

In yet another preferred embodiment, a crease 54 (FIG. 11) is made across the passageway 20. The crease may be made by sharply folding the valve at 52 (FIG. 10), preferably near the outlet port 25, and then releasing the valve. The valve will return almost to its initial planar leaving the side profile of the modified valve slightly bent as illustrated in FIGURE 10.

As in the case of the addition of a wetting agent, the crease will improve the reflux sensitivity of the valve and will restore valve function lost due to pressure, temperature and humidity changes encountered in sterilization or other procedures. The crease may be placed in the valve either before exposure to such treatments or after, although application of the crease before heating is preferred.

Finally, in a most preferred embodiment of the invention, the combination of the wetting agent and the crease have been found to significantly improve the reflux sensitivity of the valve under all conditions, including particularly when the valve is subjected to pressure, temperature and humidity changes, which would otherwise impair valve function.

## Claims

1. A one-way valve comprising two flat resilient members (12, 14) in face-to-face relationship with each other, the resilient members (12, 14) being bonded along two generally parallel tracks (16, 18) defining a passageway (20) therebetween, an inlet port (22) communicating with one end of the passageway (20), and an outlet port (25) communicating with the opposite end of the passageway (20), the passageway (20) allowing flow therethrough in a direction from the inlet port (22) to the outlet port (25) but closing in response to the application of a suction force to the inlet port (22), characterised in that the tracks (16, 18) have a tortuous profile (30) along their inner edges (26, 28) defining the passageway (20).

2. A one-way valve as claimed in claim 1, wherein the resilient members (12, 14) are made from a material chosen from the group consisting of polyethylene, mylar, nylon and polyvinyl chloride.

3. A one-way valve as claimed in claim 1, wherein each of the resilient members (12, 14) has a heat-sealable layer and a thermally-resistant layer, the resilient members (12, 14) being disposed with their heat-sealable layers in face-to-face relationship.

4. A one-way valve as claimed in claim 3, wherein the heat-sealable layer is chosen from the group consisting of low density polyethylene and ethyl vinyl acetate and the thermally-resistant layer is chosen from the group consisting of nylon, mylar and linear low density polyethylene.

5. A one-way valve as claimed in claim 3, wherein each of the resilient members (12, 14) is a laminate of low density polyethylene and nylon.

6. A one-way valve as claimed in any of the preceding claims, wherein the tortuous profile (30) comprises a sawtooth pattern.

7. A one-way valve as claimed in claim 6, wherein the sawtooth pattern comprises at least one sawtooth (30) projecting from each of the tracks (16, 18) into the passageway (20), the or each sawtooth (30) including a leading edge (32) and a trailing edge (34), the or each sawtooth (30) being oriented with its leading edge (32) directed towards the outlet port (25) and said trailing edge towards said inlet port (22).

8. A one-way valve as claimed in any of the preceding claims, including a wetting agent (50) wetting the opposing faces of the resilient members (12, 14).

9. A one-way valve as claimed in any of the preceding claims, including a crease (54) across the passageway (20).

10. A one-way valve as claimed in any of the preceding claims, wherein the valve includes greater than said two tracks (16, 18), the tracks (16, 18) defining more than one said passageway (20), the passageways (20A, 20B) having a common said inlet port (22A) and each passageway (20A, 20B) having an independent said outlet port (25A, 25B).

## Patentansprüche

1. Einwegventil, umfassend zwei flache, elastische Glieder (12, 14) in jeweils einander zugewandter Beziehung, wobei die elastischen Glieder (12, 14) entlang zweier im wesentlichen paralleler Bahnen unter Bildung eines dazwischenliegenden Durchlaßweges (20) gebondet sind, mit einem Einlaß (22), der mit einem Ende des Durchlaßweges (20) in Verbindung steht, und mit einem Auslaß (25), der mit dem gegenüberliegenden Ende des Durchlaßweges (20) in Verbindung steht, wobei der Durchlaßweg (20) einen Durchfluß in einer Richtung von dem Einlaß (22) zu dem Auslaß (25) erlaubt, aber sich bei Anwendung einer Saugkraft auf den Einlaß (22) schließt, dadurch gekennzeichnet, daß die Bahnen (16, 18) ein gekrümmtes Profil (30) entlang ihrer Innenränder (26, 28) aufweisen, die den Durchlaßweg (20) festlegen.

2. Einwegventil gemäß Anspruch 1, bei welchem die elastischen Glieder (12, 14) aus einem Material hergestellt sind, das aus der Gruppe ausgewählt ist, die aus Polyethylen, Mylar, Nylon und Polyvinylchlorid gebildet ist.

3. Einwegventil gemäß Anspruch 1, bei welchem jedes der elastischen Glieder (12, 14) eine heißversiegelbare Schicht und eine thermisch beständige Schicht aufweisen, wobei die elastischen Glieder (12, 14) mit ihren heißsiegelbaren Schichten einander zugewandt sind.

4. Einwegventil gemäß Anspruch 3, bei welchem die heißsiegelbare Schicht aus der Gruppe ausgewählt ist, die durch Polyethylen niedriger Dichte und Ethylvinylacetat besteht, und daß die thermisch beständige Schicht aus der Gruppe ausgewählt ist, die aus Nylon, Mylar und linearem Polyethylen niedriger Dichte besteht.

5. Einwegventil gemäß Anspruch 3, bei welchem jedes der elastischen Glieder (12, 14) ein Laminat aus Polyethylen niedriger Dichte und Nylon ist.

6. Einwegventil gemäß irgendeinem der vorhergehenden Ansprüche, bei welchem das gekrümmte Profil (30) eine Sägezahnkonfiguration aufweist.

7. Einwegventil gemäß Anspruch 6, bei welchem die Sägezahnkonfiguration wenigstens einen Sägezahn (30) aufweist, der von jeder der Bahnen (16, 18) in den Durchlaßweg (20) hervorsteht, wobei der oder jeder Sägezahn (30) wenigstens eine Vorderfläche (32) und eine rückwärtige Fläche (34) aufweist, und wobei der oder jeder Sägezahn (30) mit seiner Vorderfläche (32) dem Auslaß (25) zugewandt ist und mit seiner rückwärtigen Fläche dem Einlaß (22) zugewandt ist.

8. Einwegventil gemäß irgendeinem der vorhergehenden Ansprüche, umfassend ein Benetzungsmittel (50), das die einander zugewandten Flächen der elastischen Glieder (12, 14) benetzt.

9. Einwegventil gemäß irgendeinem der vorhergehenden Ansprüche, welches einen Knick (54) über den Durchlaßweg (20) umfaßt.

10. Einwegventil gemäß irgendeinem der vorhergehenden Ansprüche, bei welchem das Ventil mehr als zwei Bahnen (16, 18) umfaßt, wobei die Bahnen (16, 18) mehr als einen Durchlaßweg (20) bilden, wobei die Durchlaßwege (20A, 20B) einen gemeinsamen Einlaß (22A) aufweisen, und wobei jeder Durchlaßweg (20A, 20B) einen unabhängigen Auslaß (25A, 25B) aufweist.

## Revendications

1. Clapet de non retour comprenant deux éléments élastiques plats **(12, 14)** disposés face à face l'un avec l'autre, les éléments élastiques **(12, 14)** étant liés le long de deux bandes globalement parallèles **(16, 18)** définissant entre elles un conduit **(20),** un orifice d'entrée **(22)** communiquant avec une extrémité du conduit **(20),** et un orifice de sortie **(25)** communiquant avec l'extrémité opposée du conduit **(20),** le conduit **(20)** permettant à un écoulement de le traverser, dans le sens allant de l'orifice d'entrée **(22)** à l'orifice de sortie **(25),** mais se fermant en réponse à l'application d'une force de succion à l'orifice d'entrée **(22),** caractérisé en ce que les bandes **(16, 18)** ont un profil sinueux **(30)** le long de leurs bords intérieurs **(26, 28)** définissant le conduit **(20)**.

2. Clapet de non retour tel que revendiqué dans la revendication 1, dans lequel les éléments élastiques **(12, 14)** sont faits d'une matière choisie à partir du groupe constitué du polyéthylène, du mylar, du nylon et du chlorure de polyvinyle.

3. Clapet de non retour tel que revendiqué dans la revendication 1, dans lequel chacun des éléments élastiques **(12, 14)** comporte une couche thermosoudable et une couche résistante à la chaleur, les éléments élastiques **(12, 14)** étant disposés de sorte que leurs couches thermosoudables se trouvent face à face.

4. Clapet de non retour tel que revendiqué dans la revendication 3, dans lequel la couche thermosoudable est choisie à partir du groupe constitué du polyéthylène basse densité et de l'acétate d'éthylvinyle et dans lequel la couche résistante à la chaleur est choisie à partir du groupe constitué du nylon, du mylar et du polyéthylène basse densité linéaire.

5. Clapet de non retour tel que revendiqué dans la revendication 3, dans lequel chacun des éléments élastiques **(12, 14)** est un stratifié de polyéthylène basse densité et de nylon.

6. Clapet de non retour tel que revendiqué dans l'une quelconque des revendications précédentes, dans lequel le profil sinueux **(30)** comprend un dessin en dents de scie.

7. Clapet de non retour tel que revendiqué dans la revendication 6, dans lequel le dessin en dents de scie comprend au moins une dent de scie **(30)** faisant saillie de chacune des bandes **(16, 18)** dans le conduit **(20),** la, ou chaque, dent de scie **(30)** comportant un bord avant **(32)** et un bord arrière **(34),** la, ou chaque, dent de scie **(30)** étant orientée de sorte que son bord avant **(32)** soit dirigé vers l'orifice de sortie **(25)** et son bord arrière dirigé vers ledit orifice d'entrée **(22)**.

8. Clapet de non retour tel que revendiqué dans l'une quelconque des revendications précédentes, comprenant un agent mouillant **(50),** mouillant les faces opposées des éléments élastiques **(12, 14)**.

9. Clapet de non retour tel que revendiqué dans l'une quelconque des revendications précédentes, comportant une fronce **(54)** en travers du conduit **(20)**.

10. Clapet de non retour tel que revendiqué dans l'une quelconque des revendications précédentes, dans lequel le clapet comprend plus de deux bandes **(16, 18),** les bandes **(16, 18)** définissant plus d'un conduit **(20),** les conduits **(20A, 20B)** ayant un orifice d'entrée commun **(22A)** et chaque conduit **(20A, 20B)** ayant un orifice de sortie indépendant **(25A, 25B)**.
